# EUROPEAN PATENT APPLICATION

(11) **EP 2 292 768 A1**
(43) Date of publication of application: **09.03.2011**
(21) Application number: 10182557.8
(22) Date of filing: 03.07.2003
(51) Int. Cl.: C12N 15/54, C12N 15/60, C12N 15/82

(54) **Use of AHAS mutant genes as selection marker in potato transformation**

(30) Priority: 09.07.2002 EP 02015247
(62) Divisional of application: 10152131.8
(71) Applicant: BASF Plant Science GmbH, 67056 Ludwigshafen (DE)
(72) Inventor: Andersson, Mariette, 22229 Lund (SE); Trifonova, Adelina, 24438 Kävlinge (SE); Hofvander, Per, 23941 Falsterbo (SE)
(74) Representative: Popp, Andreas

(57) **Abstract**

The present invention relates to the use of mutated AHAS genes conferring resistance to herbicides and resulting in an highly efficient selection system for the production of transgenic potato lines.

SEQUENCE LISTING

<110> BASF Plant Science GmbH

<120> Use of AHAS mutant genes as selection marker in potato
      transformation

<130> PF53700-3

<140> 10152131.8
<141> 2003-07-03

<150> 02015247.6
<151> 2002-07-09

<160> 2

<170> PatentIn Ver. 2.1

<210> 1
<211> 5717
<212> DNA
<213> Arabidopsis thaliana

<220>
<221> CDS
<222> (2484)..(4493)

<400> 1
tctagattat gtatttccaa ctttcattaa caatataatc gcatataaat gaaaaatcgt 60

ttccaggata atattttgat gaaatctcat attattgttc gtactcggat tgatgttgaa 120

ggcttgaagc gcttcaaatt atagaccaga ttatttaagt ttttcttttg tttactccat 180

atcaatttga tccattatac tacctaagaa aatttaggta acatagaatt atttattgtt 240

atagtaaaaa aaaggaaaac cacaaaaata atctactttt acgtatatac tattttcatg 300

acataagtaa ttaagttgta caactttttt ttaatgaaaa gagagagtaa atttatcatg 360

ttcatgtgta gttacctcgt gaataaccga cggttatata gacgcctaac atgaattgtt 420

cagttgaaga cagttcaaaa catgtgtttc actctaaaat cctcaacaaa aaaaaagtgt 480

taaaatttgt aaacctcttt caagcaaaaa aagaaaaagt gttagaatcc caagattctt 540

tcataatccg gaatcttggc tgaaaacgta taaaagagat tgacgtagta acaaggagtc 600

ttggtatgct tccatgcttt ttatcctttt ttgtcatgga accatgattt ggttaccatt 660

tattatgtaa ccgaaatttt cattgtaata atgaatattt aaatttttag caaaaaaaaa 720

caaaaaaaaa caaggagtct tgtcttcgtt ctcaaatttc agagctcttg cacttttcaa 780

gagttttact ttgatgagtg agacatttgt ctttttagtg tttattttct aaacttaaaa 840

tagtagcatc aacatcactc aattataatt cttaagatgt tgtagaaaaa tattttatag 900

atggaaagta atcgatatta agacaaataa gaaaccaaac cggactttgt gttcagaccg 960

aatcaaatct gaattggaga aattatggtg gaggcgaaag tcaacggaac taaagtataa 1020

aaccaaatgt caaaaataaa acccaatttt catccttaaa cgaacctgct gaaaccctaa 1080

tttcgattac caattccgat ctaaaaagaa gtcatggaag ccattgattc cgcaatcgat 1140

cctctcagag atttcgctaa gagcagtgtt cgtctcgtcc agcgctgtca caaacccgat 1200

cgcaagggta acgccttttc tcaaaaaaat ctcatttccg atttttgatc tgtagattag 1260

ggttttctga aattttgata tcatttgtaa ttgaattggt tatcagaatt cacgaaagta 1320

gctgtgcgta cggcgattgg atttgtggtg atgggattcg ttggattctt cgtgaagctc 1380

gttttcatcc caatcaacaa catcatcgtt ggatcttctt agtgtagtac tttctttacg 1440

aggtaattga tctcgcatta tatatctaca ttttggttat gttacttgac atatagtcat 1500

tgattcaata gttctgttaa ttcctttaaa gatcattttg actagaccac attcttggtt 1560

cattcctcaa taatttgtaa tcatattggt ggatatagaa gtagattggt tatagatcag 1620

atagtggaag actttaggat gaatttcagc tagttttttt ttttggctta ttgtctcaaa 1680

agattagtgc tttgctgtct ccattgcttc tgctatcgac acgcttctgt ctccttgtat 1740

ctttattata tctattcgtc ccatgagttt tgtttgttct gtattcgttc gctctggtgt 1800

catggatgga gtctctgttc catgtttctg taatgcatgt tgggttgttt catgcaagaa 1860

atgctgagat aaacactcat ttgtgaaagt ttctaaactc tgaatcgcgc tacaggcaat 1920

gctccgagga gtaggaggag aagaacgaac caaacgacat tatcagccct ttgaggaagc 1980

tcttagtttt gttattgttt ttgtagccaa attctccatt cttattccat tttcacttat 2040

ctcttgttcc ttatagacct tataagtttt ttattcatgt atacaaatta tattgtcatc 2100

aagaagtatc tttaaaatct aaatctcaaa tcaccaggac tatgtttttg tccaattcgt 2160

ggaaccaact tgcagcttgt atccattctc ttaaccaata aaaaaagaaa gaaagatcaa 2220

tttgataaat ttctcagcca caaattctac atttaggttt tagcatatcg aaggctcaat 2280

cacaaataca atagatagac tagagattcc agcgtcacgt gagttttatc tataaataaa 2340

ggaccaaaaa tcaaatcccg agggcatttt cgtaatccaa cataaaaccc ttaaacttca 2400

agtctcattt ttaaacaaat catgttcaca agtctcttct tcttctctgt ttctctatct 2460

cttgctcatc tttctcctga acc atg gcg gcg gca aca aca aca aca aca aca 2513
Met Ala Ala Ala Thr Thr Thr Thr Thr Thr
1               5                   10

tct tct tcg atc tcc ttc tcc acc aaa cca tct cct tcc tcc tcc aaa 2561
Ser Ser Ser Ile Ser Phe Ser Thr Lys Pro Ser Pro Ser Ser Ser Lys
                15                  20                  25

tca cca tta cca atc tcc aga ttc tcc ctc cca ttc tcc cta aac ccc 2609
Ser Pro Leu Pro Ile Ser Arg Phe Ser Leu Pro Phe Ser Leu Asn Pro
            30                  35                  40

aac aaa tca tcc tcc tcc tcc cgc cgc cgc ggt atc aaa tcc agc tct 2657
Asn Lys Ser Ser Ser Ser Ser Arg Arg Arg Gly Ile Lys Ser Ser Ser
        45                  50                  55

ccc tcc tcc atc tcc gcc gtg ctc aac aca acc acc aat gtc aca acc 2705
Pro Ser Ser Ile Ser Ala Val Leu Asn Thr Thr Thr Asn Val Thr Thr
    60                  65                  70

act ccc tct cca acc aaa cct acc aaa ccc gaa aca ttc atc tcc cga 2753
Thr Pro Ser Pro Thr Lys Pro Thr Lys Pro Glu Thr Phe Ile Ser Arg
75                  80                  85                  90

ttc gct cca gat caa ccc cgc aaa ggc gct gat atc ctc gtc gaa gct 2801
Phe Ala Pro Asp Gln Pro Arg Lys Gly Ala Asp Ile Leu Val Glu Ala
                95                  100                 105

tta gaa cgt caa ggc gta gaa acc gta ttc gct tac cct gga ggt gca 2849
Leu Glu Arg Gln Gly Val Glu Thr Val Phe Ala Tyr Pro Gly Gly Ala
            110                 115                 120

tca atg gag att cac caa gcc tta acc cgc tct tcc tca atc cgt aac 2897
Ser Met Glu Ile His Gln Ala Leu Thr Arg Ser Ser Ser Ile Arg Asn
        125                 130                 135

gtc ctt cct cgt cac gaa caa gga ggt gta ttc gca gca gaa gga tac 2945
Val Leu Pro Arg His Glu Gln Gly Gly Val Phe Ala Ala Glu Gly Tyr
    140                 145                 150

gct cga tcc tca ggt aaa cca ggt atc tgt ata gcc act tca ggt ccc 2993
Ala Arg Ser Ser Gly Lys Pro Gly Ile Cys Ile Ala Thr Ser Gly Pro
155                 160                 165                 170

gga gct aca aat ctc gtt agc gga tta gcc gat gcg ttg tta gat agt 3041
Gly Ala Thr Asn Leu Val Ser Gly Leu Ala Asp Ala Leu Leu Asp Ser
                175                 180                 185

gtt cct ctt gta gca atc aca gga caa gtc cct cgt cgt atg att ggt 3089
Val Pro Leu Val Ala Ile Thr Gly Gln Val Pro Arg Arg Met Ile Gly
            190                 195                 200

aca gat gcg ttt caa gag act ccg att gtt gag gta acg cgt tcg att 3137
Thr Asp Ala Phe Gln Glu Thr Pro Ile Val Glu Val Thr Arg Ser Ile
        205                 210                 215

acg aag cat aac tat ctt gtg atg gat gtt gaa gat atc cct agg att 3185
Thr Lys His Asn Tyr Leu Val Met Asp Val Glu Asp Ile Pro Arg Ile
    220                 225                 230

att gag gaa gct ttc ttt tta gct act tct ggt aga cct gga cct gtt 3233
Ile Glu Glu Ala Phe Phe Leu Ala Thr Ser Gly Arg Pro Gly Pro Val
235                 240                 245                 250

ttg gtt gat gtt cct aaa gat att caa caa cag ctt gcg att cct aat 3281
Leu Val Asp Val Pro Lys Asp Ile Gln Gln Gln Leu Ala Ile Pro Asn
                255                 260                 265

tgg gaa cag gct atg aga tta cct ggt tat atg tct agg atg cct aaa 3329
Trp Glu Gln Ala Met Arg Leu Pro Gly Tyr Met Ser Arg Met Pro Lys
            270                 275                 280

cct ccg gaa gat tct cat ttg gag cag att gtt agg ttg att tct gag 3377
Pro Pro Glu Asp Ser His Leu Glu Gln Ile Val Arg Leu Ile Ser Glu
        285                 290                 295

tct aag aag cct gtg ttg tat gtt ggt ggt ggt tgt ttg aat tct agc 3425
Ser Lys Lys Pro Val Leu Tyr Val Gly Gly Gly Cys Leu Asn Ser Ser
    300                 305                 310

gat gaa ttg ggt agg ttt gtt gag ctt acg ggg atc cct gtt gcg agt 3473
Asp Glu Leu Gly Arg Phe Val Glu Leu Thr Gly Ile Pro Val Ala Ser
315                 320                 325                 330

acg ttg atg ggg ctg gga tct tat cct tgt gat gat gag ttg tcg tta 3521
Thr Leu Met Gly Leu Gly Ser Tyr Pro Cys Asp Asp Glu Leu Ser Leu
                335                 340                 345

cat atg ctt gga atg cat ggg act gtg tat gca aat tac gct gtg gag 3569
His Met Leu Gly Met His Gly Thr Val Tyr Ala Asn Tyr Ala Val Glu
            350                 355                 360

cat agt gat ttg ttg ttg gcg ttt ggg gta agg ttt gat gat cgt gtc 3617
His Ser Asp Leu Leu Leu Ala Phe Gly Val Arg Phe Asp Asp Arg Val
        365                 370                 375

acg ggt aag ctt gag gct ttt gct agt agg gct aag att gtt cat att 3665
Thr Gly Lys Leu Glu Ala Phe Ala Ser Arg Ala Lys Ile Val His Ile
    380                 385                 390

gat att gac tcg gct gag att ggg aag aat aag act cct cat gtg tct 3713
Asp Ile Asp Ser Ala Glu Ile Gly Lys Asn Lys Thr Pro His Val Ser
395                 400                 405                 410

gtg tgt ggt gat gtt aag ctg gct ttg caa ggg atg aat aag gtt ctt 3761
Val Cys Gly Asp Val Lys Leu Ala Leu Gln Gly Met Asn Lys Val Leu
                415                 420                 425

gag aac cga gcg gag gag ctt aag ctt gat ttt gga gtt tgg agg aat 3809
Glu Asn Arg Ala Glu Glu Leu Lys Leu Asp Phe Gly Val Trp Arg Asn
            430                 435                 440

gag ttg aac gta cag aaa cag aag ttt ccg ttg agc ttt aag acg ttt 3857
Glu Leu Asn Val Gln Lys Gln Lys Phe Pro Leu Ser Phe Lys Thr Phe
        445                 450                 455

ggg gaa gct att cct cca cag tat gcg att aag gtc ctt gat gag ttg 3905
Gly Glu Ala Ile Pro Pro Gln Tyr Ala Ile Lys Val Leu Asp Glu Leu
    460                 465                 470

act gat gga aaa gcc ata ata agt act ggt gtc ggg caa cat caa atg 3953
Thr Asp Gly Lys Ala Ile Ile Ser Thr Gly Val Gly Gln His Gln Met
475                 480                 485                 490

tgg gcg gcg cag ttc tac aat tac aag aaa cca agg cag tgg cta tca 4001
Trp Ala Ala Gln Phe Tyr Asn Tyr Lys Lys Pro Arg Gln Trp Leu Ser
                495                 500                 505

tca gga ggc ctt gga gct atg gga ttt gga ctt cct gct gcg att gga 4049
Ser Gly Gly Leu Gly Ala Met Gly Phe Gly Leu Pro Ala Ala Ile Gly
            510                 515                 520

gcg tct gtt gct aac cct gat gcg ata gtt gtg gat att gac gga gat 4097
Ala Ser Val Ala Asn Pro Asp Ala Ile Val Val Asp Ile Asp Gly Asp
        525                 530                 535

gga agc ttt ata atg aat gtg caa gag cta gcc act att cgt gta gag 4145
Gly Ser Phe Ile Met Asn Val Gln Glu Leu Ala Thr Ile Arg Val Glu
    540                 545                 550

aat ctt cca gtg aag gta ctt tta tta aac aac cag cat ctt ggc atg 4193
Asn Leu Pro Val Lys Val Leu Leu Leu Asn Asn Gln His Leu Gly Met
555                 560                 565                 570

gtt atg caa tgg gaa gat cgg ttc tac aaa gct aac cga gct cac aca 4241
Val Met Gln Trp Glu Asp Arg Phe Tyr Lys Ala Asn Arg Ala His Thr
                575                 580                 585

ttt ctc ggg gat ccg gct cag gag gac gag ata ttc ccg aac atg ttg 4289
Phe Leu Gly Asp Pro Ala Gln Glu Asp Glu Ile Phe Pro Asn Met Leu
            590                 595                 600

ctg ttt gca gca gct tgc ggg att cca gcg gcg agg gtg aca aag aaa 4337
Leu Phe Ala Ala Ala Cys Gly Ile Pro Ala Ala Arg Val Thr Lys Lys
        605                 610                 615

gca gat ctc cga gaa gct att cag aca atg ctg gat aca cca gga cct 4385
Ala Asp Leu Arg Glu Ala Ile Gln Thr Met Leu Asp Thr Pro Gly Pro
    620                 625                 630

tac ctg ttg gat gtg att tgt ccg cac caa gaa cat gtg ttg ccg atg 4433
Tyr Leu Leu Asp Val Ile Cys Pro His Gln Glu His Val Leu Pro Met
635                 640                 645                 650

atc ccg aat ggt ggc act ttc aac gat gtc ata acg gaa gga gat ggc 4481
Ile Pro Asn Gly Gly Thr Phe Asn Asp Val Ile Thr Glu Gly Asp Gly
                655                 660                 665

cgg att aaa tac tgagagatga aaccggtgat tatcagaacc ttttatggtc 4533
Arg Ile Lys Tyr
            670

tttgtatgca tatggtaaaa aaacttagtt tgcaatttcc tgtttgtttt ggtaatttga 4593

gtttctttta gttgttgatc tgcctgcttt ttggtttacg tcagactact actgctgttg 4653

ttgtttggtt tcctttcttt cattttataa ataaataatc cggttcggtt tactccttgt 4713

gactggctca gtttggttat tgcgaaatgc gaatggtaaa ttgagtaatt gaaattcgtt 4773

attagggttc taagctgttt taacagtcac tgggttaata tctctcgaat cttgcatgga 4833

aaatgctctt accattggtt tttaattgaa atgtgctcat atgggccgtg gtttccaaat 4893

taaataaaac tacgatgtca tcgagaagta aaatcaactg tgtccacatt atcagttttg 4953

tgtatacgat gaaatagggt aattcaaaat ctagcttgat atgccttttg gttcatttta 5013

accttctgta aacatttttt cagattttga acaagtaaat ccaaaaaaaa aaaaaaaaaa 5073

tctcaactca acactaaatt attttaatgt ataaaagatg cttaaaacat ttggcttaaa 5133

agaaagaagc taaaaacata gagaactctt gtaaattgaa gtatgaaaat atactgaatt 5193

gggtattata tgaatttttc tgatttagga ttcacatgat ccaaaaagga aatccagaag 5253

cactaatcag acattggaag taggaatatt tcaaaaagtt tttttttttt aagtaagtga 5313

caaaagcttt taaaaaatag aaaagaaact agtattaaag ttgtaaattt aataaacaaa 5373

agaaattttt tatatttttt catttctttt tccagcatga ggttatgatg gcaggatgtg 5433

gatttcattt ttttcctttt gatagccttt taattgatct attataattg acgaaaaaat 5493

attagttaat tatagatata ttttaggtag tattagcaat ttacacttcc aaaagactat 5553

gtaagttgta aatatgatgc gttgatctct tcatcattca atggttagtc aaaaaaataa 5613

aagcttaact agtaaactaa agtagtcaaa aattgtactt tagtttaaaa tattacatga 5673

ataatccaaa acgacattta tgtgaaacaa aaacaatatc taga 5717

<210> 2
<211> 670
<212> PRT
<213> Arabidopsis thaliana

<400> 2
Met Ala Ala Ala Thr Thr Thr Thr Thr Thr Ser Ser Ser Ile Ser Phe
1               5                   10                  15

Ser Thr Lys Pro Ser Pro Ser Ser Ser Lys Ser Pro Leu Pro Ile Ser
            20                  25                  30

Arg Phe Ser Leu Pro Phe Ser Leu Asn Pro Asn Lys Ser Ser Ser Ser
        35                  40                  45

Ser Arg Arg Arg Gly Ile Lys Ser Ser Ser Pro Ser Ser Ile Ser Ala
    50                  55                  60

Val Leu Asn Thr Thr Thr Asn Val Thr Thr Thr Pro Ser Pro Thr Lys
65                  70                  75                  80

Pro Thr Lys Pro Glu Thr Phe Ile Ser Arg Phe Ala Pro Asp Gln Pro
                85                  90                  95

Arg Lys Gly Ala Asp Ile Leu Val Glu Ala Leu Glu Arg Gln Gly Val
            100                 105                 110

Glu Thr Val Phe Ala Tyr Pro Gly Gly Ala Ser Met Glu Ile His Gln
        115                 120                 125

Ala Leu Thr Arg Ser Ser Ser Ile Arg Asn Val Leu Pro Arg His Glu
    130                 135                 140

Gln Gly Gly Val Phe Ala Ala Glu Gly Tyr Ala Arg Ser Ser Gly Lys
145                 150                 155                 160

Pro Gly Ile Cys Ile Ala Thr Ser Gly Pro Gly Ala Thr Asn Leu Val
                165                 170                 175

Ser Gly Leu Ala Asp Ala Leu Leu Asp Ser Val Pro Leu Val Ala Ile
            180                 185                 190

Thr Gly Gln Val Pro Arg Arg Met Ile Gly Thr Asp Ala Phe Gln Glu
        195                 200                 205

Thr Pro Ile Val Glu Val Thr Arg Ser Ile Thr Lys His Asn Tyr Leu
    210                 215                 220

Val Met Asp Val Glu Asp Ile Pro Arg Ile Ile Glu Glu Ala Phe Phe
225                 230                 235                 240

Leu Ala Thr Ser Gly Arg Pro Gly Pro Val Leu Val Asp Val Pro Lys
                245                 250                 255

Asp Ile Gln Gln Gln Leu Ala Ile Pro Asn Trp Glu Gln Ala Met Arg
            260                 265                 270

Leu Pro Gly Tyr Met Ser Arg Met Pro Lys Pro Pro Glu Asp Ser His
        275                 280                 285

Leu Glu Gln Ile Val Arg Leu Ile Ser Glu Ser Lys Lys Pro Val Leu

290                 295                 300

Tyr Val Gly Gly Gly Cys Leu Asn Ser Ser Asp Glu Leu Gly Arg Phe
305                 310                 315                 320

Val Glu Leu Thr Gly Ile Pro Val Ala Ser Thr Leu Met Gly Leu Gly
                325                 330                 335

Ser Tyr Pro Cys Asp Asp Glu Leu Ser Leu His Met Leu Gly Met His
                340                 345                 350

Gly Thr Val Tyr Ala Asn Tyr Ala Val Glu His Ser Asp Leu Leu Leu
        355                 360                 365

Ala Phe Gly Val Arg Phe Asp Asp Arg Val Thr Gly Lys Leu Glu Ala
    370                 375                 380

Phe Ala Ser Arg Ala Lys Ile Val His Ile Asp Ile Asp Ser Ala Glu
385                 390                 395                 400

Ile Gly Lys Asn Lys Thr Pro His Val Ser Val Cys Gly Asp Val Lys
                405                 410                 415

Leu Ala Leu Gln Gly Met Asn Lys Val Leu Glu Asn Arg Ala Glu Glu
            420                 425                 430

Leu Lys Leu Asp Phe Gly Val Trp Arg Asn Glu Leu Asn Val Gln Lys
        435                 440                 445

Gln Lys Phe Pro Leu Ser Phe Lys Thr Phe Gly Glu Ala Ile Pro Pro
    450                 455                 460

Gln Tyr Ala Ile Lys Val Leu Asp Glu Leu Thr Asp Gly Lys Ala Ile
465                 470                 475                 480

Ile Ser Thr Gly Val Gly Gln His Gln Met Trp Ala Ala Gln Phe Tyr
                485                 490                 495

Asn Tyr Lys Lys Pro Arg Gln Trp Leu Ser Ser Gly Gly Leu Gly Ala
            500                 505                 510

Met Gly Phe Gly Leu Pro Ala Ala Ile Gly Ala Ser Val Ala Asn Pro
        515                 520                 525

Asp Ala Ile Val Val Asp Ile Asp Gly Asp Gly Ser Phe Ile Met Asn
    530                 535                 540

Val Gln Glu Leu Ala Thr Ile Arg Val Glu Asn Leu Pro Val Lys Val
545                 550                 555                 560

Leu Leu Leu Asn Asn Gln His Leu Gly Met Val Met Gln Trp Glu Asp
                565                 570                 575

Arg Phe Tyr Lys Ala Asn Arg Ala His Thr Phe Leu Gly Asp Pro Ala
            580                 585                 590

Gln Glu Asp Glu Ile Phe Pro Asn Met Leu Leu Phe Ala Ala Ala Cys
        595                 600                 605

Gly Ile Pro Ala Ala Arg Val Thr Lys Lys Ala Asp Leu Arg Glu Ala
    610                 615                 620

Ile Gln Thr Met Leu Asp Thr Pro Gly Pro Tyr Leu Leu Asp Val Ile
625                 630                 635                 640

Cys Pro His Gln Glu His Val Leu Pro Met Ile Pro Asn Gly Gly Thr
                645                 650                 655

Phe Asn Asp Val Ile Thr Glu Gly Asp Gly Arg Ile Lys Tyr
            660                 665                 670

## Description

The present invention relates to an improved selection system, using a mutated acetohydroxy acid synthase (AHAS) gene, for production of transgenic potato lines. Genetic insertion of mutated AHAS genes can give plants tolerance to a number of herbicide compounds. This invention yields a higher transformation efficiency and a reduced escape rate than previously described selection systems for patato plants.

The invention relates to a method for the generation of stably transformed fertile plants of the genus *Solanum,* which comprises the following steps:
(a) growing the plant to be transformed, and obtaining the suitable explant,
(b) transferring DNA sequences into plant cells,
(c) selecting transformed plant cells and
(d) regenerating transgenic fertile plants.

Moreover, the method comprises the possibility of transferring homologous or heterologous DNA sequences into the plant to be transformed or explants thereof, for example by *Agrobacterium tumefaciens.*

Moreover, the method comprises the possibility of using leaves as explants.

Acetohydroxyacid synthase (EC 4.1.3.18; AHAS; acetolactate sythase) is an enzyme catalysing, in two parallel pathways, the first step of the synthesis of the branched-chain aminoacids valin, leucin and isoleucin. In the valin and leucin biosynthesis, AHAS is catalysing the production of acetolactate by condensation of two pyruvate molecules. While in the isoleucin biosynthesis AHAS condensates one pyruvate molecule with one 2-oxobutyrat molecule to form acetohydroxybutyrat (Umbarger, 1975). Sequence comparison of the AHAS gene in higher plants shows high conservation in at least 10 regions. These regions are probably of great importance for the AHAS function. In tobacco two unlinked genes named SuRA and SuRB code for the AHAS enzyme catalytic subunit. This is not the case in *Arabidopsis thaliana* where only one gene is coding for the enzyme.

AHAS is the target enzyme of several classes of herbicides including sulphonylureas (Ray, 1984), imidazolinones (Shaner et al, 1984), triazolopyrimidines (Subrimanian et al, 1989) and pyrimidinyl oxybenzoat (Hawkes et al, 1989). The herbicides prevent branched amino acids to be synthesised by the plant and may lead to plant death. However, mutations in the AHAS genes can result in higher tolerance to these herbicides (US 5,013,659) because of reduced affinity between enzyme and the herbicide.

In plants mutations conferring resistance to herbicides occur as a result of exposure to the compound repeatedly as it was reported for *Arabidopsis thaliana.* Once the mutated genes are isolated they can be used to genetically engineer plants for improved tolerance to the herbicides. For example mutations in the *Arabidopsis thaliana* AHAS gene have been produced and successfully confer resistance to imidazolinones as described in WO 00/26390, US 5,767,366 and US 6,225,105. Mutations in a corn AHAS gene confer imidazolinone resistance to monocot plants as described in EP 0 525 384.

The mutated AHAS genes can be used for production of herbicide resistant plants, yielding field resistance to a specific herbicide or can be used as a selection marker for genetic engineering of plants.

When genetic material is introduced into a population of plant cells, only a minor part of the cells are successfully transformed. For the production of novel genetically modified plants a selection system is transformed together with the trait genes providing the transformed cells with a selective growth advantage. This construct allows to select transformed from non-transformed cells by adding a compound favoring the regeneration of transformed shoots.

A number of selection systems have been developed; most of them are based on the use of herbicide or antibiotic resistance genes. For many plant species, several of the used selectable marker genes yield a low transformation efficiency and many non-transgenic escapes. Thus more efficient selection systems are required.

The use of antibiotic and herbicide resistance is called negative selection due to that the non-transformed cells are greatly retarded in growth or even killed. Selectable marker genes which can be used are for example the bialaphos resistance gene (bar) and the kanamycin or G418 resistance gene (NPTII). By growing the tissue on media containing for example kanamycin, in theory only the transformed cells will divide and take part in morphogenesis. The NPTII gene encodes a neomycin phosphotransferase, which reduces the inhibitory action of kanamycin, neomycin, G418 and paromomycin by a phosphorylation reaction.

In contrast to negative selection research has also been done on positive selection, based on giving the transformed cells a metabolic advantage while the non-transformed cells are starving with a concomitant slow reduction in viability. An example is the use of the mannose or xylose based selection system as described e.g. in US 5,767,378.

*Solanum tuberosum* is one of the major target crops for genetic engineering. Main traits for potato are tuber quality, nutritional composition, starch quality, starch yield, insect and virus resistance. One trait with high market potential is the production of high amylopectin starch. The high amylopectin starch has an improved performance in the adhesive and paper industry compared to ordinary starch. In the paper production it will be used as a binder and for coating with printing quality better than latex used today.

Solanum tuberosum is a tetraploid plant with a high level of genetic heterozygosity. Conventional breeding of potato is therefore complicated because of segregation of important characteristics. Genetic engineering has during the last decade been an alternative to conventional breeding when it comes to improving potato varieties. A single trait or a combination of traits are more efficiently introduced by transformation than by using conventional breeding. Due to the fact that genetically modified potato plants can efficiently be vegetatively propagated the trait is fixed in the commercial line without the normal subsequent breeding.

Agrobacterium tumefaciens mediated transformation has successfully been achieved in potato since 1986. Almost exclusively the nptII gene has been used as a selection marker in potato.

The highest documented efficiency using nptII as selection marker in potato transformation is described by Libiakova, G. et al. in 2001. They have produced 120 leaf discs. Of those 81% produced one or more shoots. 90% of the shoots were confirmed transformants and showed resistance to kanamycin. This is equivalent to a regeneration efficiency of 81%, an escape rate of 10% and yields a transformation efficiency of 73%.

It is an object of the present invention to provide an efficient method for transformation of potato plants with a low escape rate and a high transformation efficiency.

Leaf segments are the explants of choice used in Agrobacterium mediated potato transformation. Transgenic plants are regenerated via direct or indirect organogenesis under selective conditions. Cytokinines as growth hormones are mainly involved in provoking cell division and adventitious bud formation in in vitro potato leaf segments. When Imazamox - (RS)-2-(4-isopropyl-4-methyl-5-oxo-2-imidazolin-2-yl)-5-methoxymethylnicotinic acid, an imidazolinone type herbicide - is applied in low concentrations it was surprisingly found that it possesses a cytokinine-like effect in tissue culture, increasing plant cell growth. At higher concentrations the substance is acting as an herbicide. The invention shows that Imazamox can efficiently be used as a selective agent in the recovery of transgenic potato plants and as a cytokinine- like substance it is supporting regeneration during transformation. This phenomenon classifies Imazamox as a unique selection agent that is combining both lethality for non-transgenic cells and improved growth for transgenic cells.

The present invention is describing a new selection system for efficient recovery of transgenic plants. The invention is also describing an innovative selection system that is acting as restriction for untransformed cells and support for transformed ones. Finally the invention is presenting an efficient system for recovery of transgenic potato plants and yields only a very small escape rate.

The present invention relates to the use of a mutated AHAS gene conferring imidazoline type herbicide resistance resulting in a highly efficient selection system for production of transgenic potato lines.

The invention provides a key advantage by minimizing the escapes to almost zero of all shoots regenerated without interfering with the high number of shoots regenerated per explant.

Another advantage is that the method does not comprise a gene conferring resistance to an antibiotic.

For example a mutated gene coding for a functional AHAS enzyme with tolerance to the imadazolinone type herbicides as described in Sathasiavan, K. et al, 1991 is used, see SEQ-ID No. 1. The mutated AHAS gene is used for insertion as a selection marker together with any other homologous or heterologous gene into the potato genome for production of transgenic potato plants. The AHAS enzyme naturally exists in higher plants and the use of a modified plant AHAS gene as selection marker conferring resistance to AHAS inhibiting herbicides does not result in the addition of a new biosynthetic function.

Furthermore
(i) the mutated AHAS gene can be of eukaryotic or prokaryotic origin;
(ii) the mutated AHAS gene can be regulated by any promoter functional in plants;
(iii) any plant transformation method for insertion of the gene constructs can be used;
(iiii) any mutations in the AHAS gene resulting in tolerance to an AHAS inhibiting herbicide can be used;
(iiiii) any potato variety can be transformed.

A mutated AHAS gene with mutation S653N as described in SEQ-ID No. 1 and US 5,767,366 was cloned in a vector with the reporter gene beta-glucuronidase (GUS) (Jefferson, R.A. et al., 1987) and transformed to potato cells, see example 1 to 4. The GUS activity is tested in the transgenic lines produced and results in a transformation frequency of 93 to 100% of all transgenic shoots analysed. The transformation method produces a very high amount of shoots per explants, 3 to 5 independent transgenic shoots dependent on the variety used. With a calculation on transformation efficiency per explants the number can be as high as 500%. This is the highest transformation frequency of potato published. The mutated AHAS gene has also been used in combination with a gbss antisense gene for production of high amylopectin potato lines, see example 12. The AHAS selection system yielded in this case the same high transformation efficiency, which establishes the use of the AHAS gene as a selection marker also for commercially important traits.

The highest efficiency of *nptII* as a selection marker is 74% (see example 10) of totally analyzed shoots yielding a transformation efficiency of 370% at the maximum. Another selection system that can be used for potato transformation is the Mannose or xylose based selection system. Haldrup et al. (1998) have used the xylose isomerase gene in potato transformation, which yielded a transformation efficiency of 29% based on number of explants producing GUS positive shoots. High percentage of shoots positively transformed has been published for sugar beet and tobacco when using a very high mannose concentration, however the high concentration of the selective agent inhibit the number of shoots per explant distinctively (Joersbo et al., 1998).

The process furthermore comprises the possibility that compounds with herbicidal activity inhibiting the AHAS synthase can be employed for the selection of transgenic potato plants by using different combinations of growth regulators as for example auxins, cytokinines and/or auxin or cytokinines conjugates for the regeneration into intact transgenic plants.

The method is suitable for transforming the species Solanum tuberosum.

The invention also relates to transgenic fertile *Solanum* plants themselves which have been generated by the method described and to their transgenic seeds.

The method according to the invention allows for the first time to transform plant cells of the genus *Solanum* with a transformation efficiency of more than 93% to be regenerated into transgenic plants as shown in example 9.

The method for the generation of stably transformed *Solanum* plants is composed of the following steps: (a) growing the plant to be transformed and obtaining the suitable explant, (b) transferring DNA sequences into plant cells, (c) selecting transformed plant cells with compounds which inhibit in non transformed plant cells the AHAS synthase and (d) regenerating these transformed plant cells into fertile transgenic plants.

A variety of methods is currently available for the transformation. The most frequently employed method for transforming dicotyledonous plants is the *Agrobacterium-tumefaciens-*mediated gene transfer. This method exploits the natural ability of the soil bacterium to integrate genetic material into the plant genome. Other suitable methods are for example protoplast transformation by polyethylene-glycol-induced DNA uptake, electroporation, sonication or microinjection, the transformation of intact cells or tissue by micro- or macroinjection into tissue or embryos, tissue electroporation, incubation of dry embryos in DNA-containing solution, vacuum infiltration of seeds and the biolistic gene transfer.

The use of *Agrobacterium tumefaciens* for the transformation of plants using tissue culture explants has been described by Horsch et al., Science 228(1985), 1229-1231; Fraley et al., Proc. Natl. Acad. Sci. USA 80(1983), 4803-4807 and Bevans et al., Nature 304(1983), 184-187. Many strains of *Agrobacterium tumefaciens* are capable of transferring genetic material into *Solanum* species such as for example the strains EHA 101, EHA105, LBA4404 and C58C1 with various disarmed Ti-plasmids.

The agrobacterial strain employed for the transformation of potatoes contains in addition to its disarmed Ti-plasmid a binary plasmid with the T-DNA to be transferred which contains a gene for selecting the transformed cells and the gene to be transferred. Both genes must be equipped with transcriptional and translational initiation and termination signals. The binary plasmid can be transferred into the agrobacterial strain for example by electroporation or other transformation methods, see Mozo & Hooykaas, Plant Mol. Biol. 16(1991), 917-918. Coculture of the plant explants with the agrobacterial strain takes two to three days.

Foreign genes can be expressed in a constitutive, inducible (by biotic and abiotic factors), tissue-specific or development-specific way. A relatively constitutive expression is achieved for example in plants by the cauliflower mosaic virus 35S promoter. This expression has been described by Shewmaker et al., Virology 140, 281-288 (1985) and Gardner et al., Plant Mol. Biol. 6, 221-228(1986).

Both the direct and the indirect gene transfer are suitable transformation methods. The *Agrobacterium-mediated* transformation using a wide range of starting explants such as for example cotyledons, leaves, hypocotyls, shoots, roots, callus, mature and immature seeds or floral tissue can successfully be employed. The gene transfer can be effected both by simple cocultivation/incubation or wetting with the agrobacterial strain and by a supporting vacuum infiltration of the explants with the bacterial culture in question. The use of feeder cultures as aids may be advantageous in the process. Each agrobacterial strain which contains a Ti- or Ri-plasmid with the genetic information required for the transfer is suitable as vector for the transformation. Suitable agrobacterial strains are for example EHA101 [pEHA101], EHA105[pEHA105], LBA4404[pAL4404], C58C1 [pMP90] and C58C1 [pGV2260]. Viral vectors also seem to be suitable for the transformation of *Solanum.* Other methods for transferring genetic material into *Solanum* are for example the polyethylene glycol (PEG)-mediated protoplast transformation, the electroporation, the sonication or microinjection and the transformation of intact cells or tissue by micro- or macroinjection into tissue or embryos, tissue electroporation, incubation of dry embryos in DNA-containing solution, the vacuum infiltration of seeds, see also Eds. Galun, E. and Breiman, A. In: Transgenic Plants, Imperial College Press, 1997. The use of the particle gun for bombarding a wide range of explants such as for example leaves and callus, is also possible.

All gene transfer vectors which contain the border sequences necessary for transferring the T-DNA (left and right border, LB and RB respectively), carry one or more selectable marker or reporter genes under the control of suitable promoters and terminators and/or contain further useful genes or target genes, also under the control of suitable transcriptional and translational regulatory units, can be employed for the Agrobacteria-mediated transformation. The transfer of DNA sequences into the *Solanum* plant to be transformed or explants thereof is preferably done by *Agrobacterium-tumefaciens-mediated* gene transfer.

The regeneration medium according to the invention furthermore comprises growth regulators such as auxins and/or auxin conjugates, and cytokinins and/or cytokinin conjugates. Suitable auxins are both natural and synthetic auxins. The natural auxin is indoleacetic acid (IAA), examples of synthetic auxins are 3-indolebutyric acid (IBA), 1-naphthylacetic acid (NAA) and the herbicide 2,4-dichlorophenoxyacetic acid (2,4-D). Other herbicides which act as auxins are also feasible as growth regulators. Auxin conjugates are compounds of auxin (IAA) with aspartic acid, glucose, myoinositol and others. In the case of the cytokinins, again, natural cytokinins such as, for example, zeatin and synthetic components, such as 6-benzylaminopurine (BAP) and 6-furfurylaminopurine (kinetin) may also be employed. Zeatin riboside is frequently employed as cytokinin conjugate. The auxins and the cytokinins can be employed in each case as individual components, but also as auxin or cytokinin mixtures. The concentration of the individual growth regulators is 0.05 to 10 mg/l, preferably 0.1 to 5 mg/l.

The transformation method according to the invention can be applied, inter alia, to Solanaceae species as Solanum tuberosum (potato), Lycopersicon esculentum (tomato) and pepper.

The mutant AHAS genes of the present invention confer resistance to imidazolinone herbicides. Types of herbicides to which resistance is conferred are described for example in US Patent Nos.: 4,188,487; 4,201,565; 4,221,586; 4,297,128; 4,554,013; 4,608,079; 4,638,068; 4,747,301; 4,650,514; 4,698,092; 4,701,208; 4,709;036; 4,752;323; 4,772,311 and 4,798,619.

Mutant AHAS genes conferring resistance to AHAS inhibiting herbicides are described in US 5,013,659, US 5,141,870 and US 5,378,824.

Other mutant AHAS genes of the present invention could also confer resistance to sulfonylurea herbicides. Types of mutants which confer sulfonylurea resistance are described for example in US 5,853,973 and US 5,928,937.

Mutant AHAS genes conferring resistance to imidazolinone type herbicides are described in WO 00/26390 and US 5,767,366.

Furthermore Duggleby, R.G. and Pang, S.S. in Journal of Biochemistry and Molecular Biology 33(1), 1-36 (2000) describe mutations of the AHAS genes which could be used in the invention for conferring herbicide resistance to transgenic potato plants.

In WO 00/26390 additional genomic and cDNA sequences coding for an eukaryotic AHAS small subunit protein are disclosed. The DNA sequences and vectors are used to transform plants to produce transgenic plants which possess elevated levels of tolerance or resistance to herbicides such as imidazolinones.

It will be understood by those working in the field that the nucleic acid sequence depicted in SEQ-ID No. 1 is not the only sequence which can be used to confer imidazolinone-specific resistance. Also contemplated are those nucleic acid sequences which encode an identical protein but which, because of the degeneracy of the genetic code, possess a different nucleotide sequence. The invention also encompasses genes encoding AHAS sequences in which the above-mentioned mutation is present, but which also encode one or more silent amino acid changes in positions of the molecule not relevant for resistance to herbicides or to the catalytic function. Also contemplated are gene sequences from other imidazolinone resistant monocot or dicot plants which have a mutation in the corresponding region of the sequence.

For example, alterations in the gene sequence which results in the production of a chemically equivalent amino acid at a given site are contemplated; thus, a codon for the amino acid alanine, a hydrophobic amino acid, can readily be substituted by a codon encoding another hydrophobic residue, such as glycine, or may be substituted with a more hydrophobic residue such as valine, leucine or isoleucine. Similarly, changes which result in a substitution of a negatively charged residue for another, such as aspartic acid for glutamic acid, or one positively charged residue for another, such as lycine for arginine, can also be expected to produced a biologically equivalent product.

The invention also encompasses chimaeric genes, in which the substituted portion of the corn or Arabidopsis AHAS gene is recombined with unaltered portions of the normal AHAS gene, in which the substituted portion of the corn or Arabidopsis AHAS gene is recombined with unaltered portions of the normal AHAS gene from other species. Thus, throughout the specification and claims, wherever the term " herbicide resistant AHAS gene" is used, it is intended to cover each of these alternate embodiments as well as the sequence of SEQ-ID No. 1.

For expression of the mutated AHAS gene conferring herbicide resistance in potato the following promoters can be used:
the tuber specific gbss-promoter from potato described in WO 92/11376, and the patatin promoter described in Rocha-Sosa et al., 1989 EMBO J. 8:23-29;
the light inducible promoter: cytosolic FBPase from potato described in WO 98/18940;
the octopine promoters (US 5,428,147); the triple OCS enhanced vATPase c1 promoter from Beta vulgaris (Plant Mol Biol (1999) 39: 463-475);
Constitutive promoters: for reference see Benfey et al., EMBO J. 8 (1989), 2195-2202; the 35S promoter (Franck et al., Cell 21, (1980), 285-294) or enhanced versions; the 19S promoter, see US 5,352,605 and WO 84/02913;
the RUBISCO small subunit SSU promoter: see US 4,962,028;
plastid specific promoter: see e.g. the RNA polymerase promoter in WO 95/16783 and WO 97/06250 or the clpP-promoter in WO 99/46394;
the AHAS promoter as described in US 6,025,541;

Other promoters for the expression of genes in the leaf, in the callus, in specific tissues as e.g. in the tubers or other parts of the potato plant could also be used.

The invention can especially be carried out by using the AHAS promoter, the AHAS resistance gene S653N as described in US 5,767,366 and the AHAS terminator from Arabidopsis thaliana cut from pAC321 as described in example 3.

The Arabidopsis AHAS gene (S653N) used for transformation and selection contains most of the common restriction sites for cloning such as HindIII, BamHI, EcoRI, Sstl, BgIII and EcoRV. Alteration of the molecular composition of the AHAS gene can be performed to eliminate restriction sites without altering the amino acid sequence of the resulting protein. Special care must be taken not to alter the codon usage profile from the one found in Arabidopsis, which could lead to reduced translation efficiency.

The designed gene can be made synthetically and accommodates suitable restriction sites at 5' and 3' ends for cloning.

Comparative studies have to be performed in potato in order to confirm that the synthetically made gene does not differ from the original mutated AHAS gene in conferring herbicide resistance.

For selection of transgenic potato plants chemical compounds inhibiting the AHAS enzyme can be used. Usefull compounds are the imidazoline type herbicides. Especially useful compounds are selected from the group consisting of imazethapyr (Pursuit™), imazamox (Raptor™), imazamethabenz (Assert™), imazapyr (Aresenal™), imazapic (Cadre™) and imazaquinon (Scepter™)_{.}

For selection of transgenic plants chemical compounds as described in the review article by Duggleby, R.G. and Pang, S.S. in Journal of Biochemistry and Molecular Biology 33(1), 1-36 (2000) can be used.

The mutated AHAS gene as described in SEQ-ID No. 1 has been established as a selection marker in five different potato varieties, see table 2 and 3, and can thus be used for transformation of potato varieties in general. Two different selection pressures, using 0.3 and 0.5 µM Imazamox, have been tested, see table 2. For selection on 0.3 µM Imazamox the transformation efficiency is ranging from 7 to 76 % depending on the variety used. When increasing the selection to 0.5 µM Imazamox the transformation rate is increasing to 93 to 100% respectively. The high transformation efficiency at 0.5 µM Imazamox is independent from the variety used. The fifth variety Seresta has as can be seen in table 3 and 4 a transformation efficiency of 98 to 100% at 0.5 µM Imazamox.

The invention furthermore relates to a plant expression vector comprising SEQ-ID No. 1 wherein the heterologeous DNA sequence encodes a peptide, protein, antisense-, sense-RNA, viral RNA or ribozyme.

The invention furthermore relates to a plant expression vector comprising SEO-ID No. 1 wherein the heterologeous DNA sequence contains information that causes changes in the carbohydrate concentration and the carbohydrate composition of regenerated potato plants.

The invention furthermore relates to a plant expression vector comprising SEO-ID No. 1 wherein the heterologeous DNA sequence contains information that causes the increased production of amylose type starches by using gene constructs as described in WO 92/11375, WO 92/14827, WO 96/34968 and WO 97/20040.

The invention furthermore relates to the use of a DNA sequence SEO-ID No. 1 or a DNA sequence comprising a nucleotide sequence which hybridizes to a complementary strand of the nucleotide SEO-ID No. 1 or a DNA sequence comprising a nucleotide sequence which is degenerated to the nucleotide sequence SEQ-ID No. 1 or a DNA sequence being a derivative, analogue or fragment of a nucleotide sequence encoding a protein possessing AHA synthase activity and conferring resistance to AHA synthase inhibitors.

The experiment shown in example 12 with the high amylopectin trait establishes the use of the AHAS resistance gene conferring resistance to herbicides inhibiting the AHAS enzyme as a selection marker also for commercially important traits. The transformation efficiency is 98 and 100% respectively. Analysis of the amylopectin/amylose ratio shows that in the transgenic shoots selected the amylopectin content is higher than in non transgenic control shoots. This is proof that by using the invention as described commercially relevant transgenic potato plants can be generated.

Furthermore the invention is disclosed as follows:
A) A method for transformation of potato plants by transforming potato plant cells with an expression vector comprising
   a) regulatory sequences of a promoter active in plants;
   b) operably linked thereto a DNA sequence encoding a protein with the biological activity of an AHA synthase resistant to inhibitors of potato plant wildtype AHA synthase; and
   c) operably linked thereto regulatory sequences which serve as transcription termination and/or polyadenylation signals in plants,
   selecting for AHA synthase inhibitor resistant cells and regenerating them to transgenic plants.
B) A method for transformation according to A, wherein the expression vector comprises a DNA sequence according to SEO-ID No. 1.
C) A method for transformation according to A, wherein the DNA sequence encoding a protein with the biological activity of an AHA synthase resistant to inhibitors of potato plant wildtype AHA synthase is selected from the group consisting of
   a) DNA sequence comprising a nucleotide according to SEO-ID NO. 1;
   b) DNA sequence comprising a nucleotide sequence which hybridizes to a complementary strand of the nucleotide sequence a)
   c) DNA sequence comprising a nucleotide sequence which is degenerate to the nucleotide sequence of a) and
   d) DNA sequence being a derivative, analogue or fragment of a nucleotide sequence of a), b) or c) and encoding a protein possessing AHA synthase activity and conferring resistance to AHA synthase inhibitors.
D) A method for transformation according to A to C, wherein the AHA synthase promoter from Arabidopsis thaliana or the nos promoter is used.
E) A method for transformation according to A to D, wherein the AHA synthase terminator from Arabidopsis thaliana or the OCS terminator is used.
F) A method for transformation according to A to E, wherein for selection a imidazolinone type herbicide is used.
G) A method for transformation according to F, wherein for selection (RS)-2-(4-isopropyl-4-methyl-5-oxo-2-imidazolin-2-yl)-5-methoxymethylnicotinic acid is used.
H) A plant expression vector according to A or B additionally comprising a heterologous DNA sequence.
I) A plant expression vector according to H, wherein the heterologous DNA sequence encodes a peptide, protein, antisense-, sense-RNA, viral RNA or ribozyme.
J) A plant expression vector according to I, wherein the heterologous DNA sequence contains information that causes changes in the carbohydrate concentration and the carbohydrate composition of regenerated potato plants.
K) A plant expression vector according to J, wherein the heterologous DNA sequence contains information that causes the increased production of amylopectin type starches.
L) A plant expression vector according to J, wherein the heterologous DNA sequence contains information that causes the increased production of amylose type starches.
M) A transgenic potato plant cell produced by the method for transformation according to any of A to G and containing a plant expression vector according to H to L.
N) A transgenic potato plant produced by the method of transformation according to A to G, wherein the regenerated plant exhibits an elevated resistance to imidazolinone type herbicides.
O) Harvest product of the transgenic potato plant according to M and N comprising a DNA sequence SEQ ID No. 1 or a DNA sequence according to C.
P) Harvest product according to O wherein the harvest product is a tuber.
Q) Propagation material of transgenic potato plants comprising a DNA sequence SEQ ID No. 1 or a DNA sequence according to C.
R) Use of a DNA sequence SEQ ID No. 1 according to B or a DNA sequence according to C, or a plan expression vector according to H to L in potato plant cells, potato tissue cultures, potato plants and/or potato plant breeding.

The invention now having been generally described will be more readily understood by reference to the following examples, which are included for the purpose of illustration only, and are not intended to limit scope of the present invention.

### Example 1

### Test for transgenicity - Detection of glucuronidase expression

Microtubers of the regenerated in vitro potato plants were subjected to a qualitative glucuronidase (GUS) enzyme detection by infiltrating cut microtubers for 1 minute in vacuo with the GUS substrate 5-bromo-4-chloro-3-indolyl-ß-D-glucuronic acid (X-GlcA) in a 100 mM sodium phosphate buffer, pH 7.0, which contained 10 mM EDTA, 0.1% Triton X100 and 10 mM DTT, and subsequently incubating them for approximately 15 hours at 37°C. Microtubers showed an intense blue coloration, which proves that the reporter gene is expressed in *Solanum.*

### Example 2

### Investigation of selection pressure

Potato leaf segments from in vitro propagated plants of four different potato varieties Prevalent, Producent, Karnico and Desiree were tested for natural tolerance on different concentrations of the imidazolinone herbicide Imazamox. It is important to choose a selection concentration at which the potato leaf tissue is viable long enough to regenerate shoots but have a high enough concentration to prevent the regeneration of untransformed shoots. Doses 0.1, 0.5, 1, 5, 10, 20, 30 and 50 µM Imazamox were tested.

Fully expended potato leaves are diagonally cut in 2 pieces and precultivated on MC-plates for 2 to 3 days at 23 to 24°C, see table 1.

The leaf tissues are transferred on MS300 medium for additional 2 days and cultivated under modest light at 23 to 24°C simulating a co-cultivation step. Subsequently the leaf segments are moved to MS400 plates containing 400mg/l Claforan. Claforan is added in order to suppress growth of *Agrobacterium tumefaciens.* Therefore the regeneration of shoots on different concentrations of Imazamox need to be monitored in presence of Claforan. Selection is taking place within 4 to 5 days when the explants are moved to MS400 medium supplemented with 400mg/l Claforan and Imazamox at above concentrations.

The explants are transferred to fresh selection medium every fortnight.

**Table 1: Media used for potato transformation.**

| | |
|---|---|
| MC plates | MS300 |
| | |
| MS300 plates with 1.5-2 ml liquid MS100 medium and covered with one sterile filter paper | X MS-medium 2mg/l NAA (naphtyl acetic acid) 1mg/l BAP (6-benzyl amino pyridine) 3% (w/v) sucrose pH 5.2 |
| MS 10 | MS400 |
| | |
| 4.4 g/l MS-medium (Murashige and Skoog) 1% (w/v) sucrose pH 5.8 | 4.4g/l MS-medium 2mg/l zeatine 0.01mg/l NAA (naphtyl acetic acid) mg/l GA3 (giberellic acid) 10% (w/v) sucrose 400 mg/l claforan Imazamox or kanamycin pH5.8 |
| MS 30 | Microtuber medium |
| | |
| 4.4 g/l MS-medium | 4.4 g/l MS-medium |
| 3% (w/v) sucrose | 2.5 mg/l kinetin |
| pH 5.8 | 0.5 mg/l ABA (abscisic acid) |
| | 8% sucrose |
| | 200 mg/l claforan |
| MS100 | |
| | |
| 4.4 mg/l MS-medium | |
| 30g/l sucrose | |
| 0.5 mg/l thiamin-HCl | |
| 0.5 mg/l pyridoxin-HCl | |
| 1 mg/l nicotinacid | |
| 0.5 mg/l kinetin | |
| 29.8 mg/l ferrous sulfate hepta hydrate | |
| 1 mg/l 2,4-Dichlorophenoxyacetic acid | |
| 2 g/l caseinhydrolysate | |
| pH 5.2 | |

### Example 3

### Construction of binary vectors pAHASGUS, pAP1 and pAP2

For all constructs the same AHAS mutant gene with mutation S653N originating from *Arabidopsis thaliana* was used (Sathasivan, K. et al., 1991).

### pAHASGUS

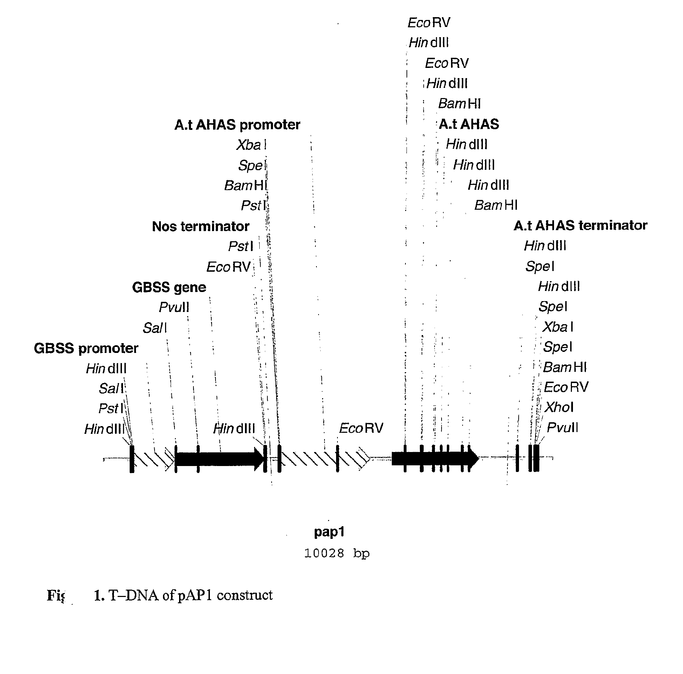

AHAS gene with mutation S653N as described in SEQ ID No. 1, originating from *Arabidopsis thaliana,* was used together with the nos promoter (Herrera, L. et al., 1983) and the OCS terminator (Wesley S. V. et al., 2001) in the binary vector pGPTVkan (Becker, D. et al., 1992). The pBIN19 based pGPTVkan was digested with Apal and BamHI discarding the *nptII* gene. The remaining 12600 bp contained from right border the nos terminator, the uidA gene (also known as GUS), the cloning cassette, the nos promoter and the pAg7 terminator. The AHAS gene with mutation S653N and the OCS terminator was ligated to the digested pGPTVkan and named pABA1, see figure 1.

For expression of the GUS gene a tuber specific gbss promoter 988bp (WO 92/11376) was cloned at the Smal site of pABA1.

The resulting pAHASGUS construct was used for *Agrobacterium tumefaciens* transformation of potato, where regenerated plantlets were analysed for GUS expression to determine the transformation efficiency.

### pAP1

AHAS gene with mutation S653N, driven by the *Arabidopsis thaliana* AHAS promoter as described in US 5,750,866, was used as a selection gene for co-transformation with a granule-bound starch synthase (gbss) antisense gene (EP-A 0 563 189).

A 293 bp nos terminator was cut out from pHAxwO and ligated to pBluescript with EcoRI. The 2952 bp gbss promotor and gbss gene in antisense direction (EP-A 0 563 189) was cut from pHAxwO and ligated in front of the nos terminator with Hindlll resulting in pMJ2. The gbss complex was cut out from pMJ2 with Xbal-Xhol and ligated to pSUN1 (WO 02/00900) cut with Xbal-Sall resulting in the construct named pMJ3.

pAC321 was cut with Xbal which yielded a fragment of 5717 bp containing AHAS promoter, AHAS gene and AHAS terminator all originating from *Arabidopsis thaliana* as described in SEQ-ID No. 1.

SEQ-ID No.1 contains the following elements:
1-2483 At AHAS promoter
2484-4496 At AHAS gene
4497-5717 At AHAS terminator

The pAC321 fragment was ligated to pMJ3 opened with Xbal.

The resulting pAP1 (figure 1) construct was used for *Agrobacterium tumefaciens* transformation of potato, which were analysed for high amylopectin starch quality.

### pAP2

AHAS gene with mutation S653N, driven by the nos promoter (US 6,174,724), was used as a selection marker for co-transformation with a gbss antisense gene (WO 92/11376).

A nos promotor of 600 bp was cut out from pGPTVKan (Becker et al., 1992) with HindIII, blunted and cut with BgIII. The fragment was ligated to pBluescript (Stratagene) cut with Spel, blunted and cut with BamHI. The construct was named pPnos2.

A 2986 bp fragment of pACGH101 containing the AHAS gene with mutation S653N as described in SEQ ID No. 1 and OCS terminator was cut out with Pstl and Pvull. The AHAS gene originates from *Arabidopsis thaliana* see pAP1. The pACGH101 fragment was ligated to pPnos2 cut with Pstl and EcoRV and named pPnoas.

The 3406 bp AHAS complex was cut out from pPnoas with Xbal-PvuII and ligated to pMJ3 (see pAP1) cut open with Xbal.

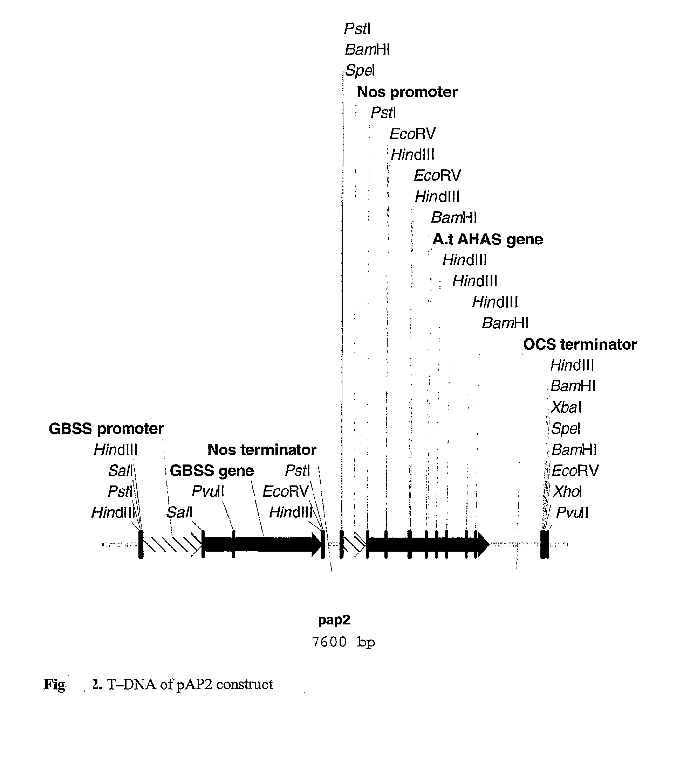

The resulting pAP2 (figure 2) construct was used for *Agrobacterium tumefaciens* transformation of potato, which were analysed for high amylopectin starch quality.

### Example 4

### Transformation method

Fully expanded leaves from in vitro propagated potato plants are diagonally cut in 2 pieces and precultivated on MC-plates for 2 to 3 days at 23 to 24°C.

Agrobacterium tumefaciens containing pAHASGUS is grown in YEB medium containing 25 µg/ml kanamycin and 100 µg rifampicin and grown over night on constant shaking (200 rpm) at 28°C.

The Agrobacterium culture is prepared for infection by dilution 1:20 with MS10 medium. The leaf explants are infected for 8-10 min in the bacterial solution and afterwards drained on filter paper for 5 to 20 seconds. The leaf segments are placed on the MS300 plates for 2 days co-cultivation under modest light at 23 to 24°C. At the end of co-cultivation the leaf segments are moved to M400 plates containing 400 mg/l Claforan to suppress bacterial growth. After 4 to 5 days the explants are moved to selection medium MS400 supplemented with 400 mg/l Claforan and Imazamox at the previously detected concentrations of 0.3 or 0.5 µM.
Leaf segments are transferred to fresh MS 400 selection medium every fortnight. The regenerated putative transgenic shoots are collected and cultivated on MS30 plates with 200mg/l Claforan aiming at shoot elongation.

When the shoots are 3 to 5 cm long, 1 to 2 cm are cut off and grown on microtuber medium in the dark at 25°C. After 2 to 5 weeks microtubers are produced. Putative transgenic plants are analysed for GUS expression in microtubers to determine the transformation efficiency.

### Example 5

### GUS expression analysis for determination of the transformation efficiency

A thin slice from each microtuber was placed in a microtiter plate. The slices were incubated with 1 mM X-gluc in 50 mM NaH₂PO₄ pH 7.2 at 37°C for approximately 1 hour. A very distinct blue colour appeared on tuber slices if a successful integration and expression of the GUS gene was achieved.

### Example 6

### Transformation efficiency determination of pAP1 and pAP2 by PCR analysis

Transgenic shoots were distinguished from non-transgenic shoots - for example escapes - by using PCR. DNA was extracted according to DNeasy 96 Plant protocol (Qiagen, Germany). In a 96 well microtiter plate, 10 to 15 mg leaf tissue was added to each well together with a 5 mm steel ball each well then representing one individual shoot. The plates were frozen in N₂(I) before homogenisation. The homogenisation was done at 30 Hz in a Mixermill 300 for 1 min. The DNA was at the end of the extraction protocol eluted in 75 ml H₂O.

Specific primers were used to amplify a fragment of the mutant S653N AHAS gene. Successful integration of the mutant AHAS gene results in a fragment of 509 bp upon PCR amplification using the specific primers.
Forward primer: AHAS1_frw: AACAACAACATCTTCTTCGATC
Reverse primer: AHAS1_rev: TAACGAGATTTGTAGCTCCG

The PCR reactions were done with the extracted DNA setup and run as follows:

### Reaction:

| | |
|---|---|
| 10x PCR Mix | 2,0 µl |
| Primer frw (25 µM) | 0,4 µl |
| Primer rev (25 µM) | 0,4 µl |
| dNTPs (10 mM) | 0,4 µl |
| RedTAQ (Sigma) | 1,0 µl |
| Templat (-20 ng/µl) | 4,0 µl |
| H₂O | 11,8 µl |

### PCR program:

| | |
|---|---|
| 94°C | 30 s |
| 59°C | 30 s x 29 cycles |
| 72°C | 30 s |
| 72°C | 7 min |
| 8°C | Hold |

Negative and positive control was included in all runs.
The reactions were analysed on 1.5% agarose gels.

### Example 7

### Starch quality analysis of amylopectin/amylose ratio

The screening for an altered amylopectin/amylose ratio was performed on transgenic lines selected for high quality integration of the intended genetic insert. The screening was done by staining with iodine (Lugol' s solution: (6.7 g/l KI + 3.3 g/l I₂) and (glycerol); ratio 1:1). Iodine stains starch containing amylose blue and starch exclusively containing amylopectin red-brown. A microtuber was crushed and a few drops of Lugol' s solution were added. The starch amylopectin quality was analysed under the microscope according to the staining colour of the starch.

### Example 8

Leaf tissues were tested for the use of the AHAS resistance gene contained in SEQ ID No. 1 and for the selection of transgenic potato plant cells as described above. Four different potato cultivars Prevalent, Producent, Karnico and Desiree were cultivated on different concentrations of Imazamox, an imidazolinones type herbicides. It is essential to use the proper concentration for selection to yield an acceptable number of shoots per explant without high escape rate. The lethality of various Imazamox concentrations on regeneration was evaluated.

Lethal dose for regeneration was detected within 0.1 to 1 µM ranges of concentrations. Slight variation in lethality was detected between varieties. No regeneration occurs when 0.5 µM Imazamox was included in the medium. The concentrations 0.3 µM and 0.5 µM are selected for transformation experiments providing a lethality of 80 and 100% in regeneration.

### Example 9

Transformation of four different potato varieties (Prevalent, Producent, Kuras and Desiree) was performed with pAHASGUS as described above. For selection a concentration of 0.3 or 0.5 µM Imazamox was used. Each shoot was analysed according to GUS activity and the number of GUS positive shoots in total is listed in table 2. A distinct blue colour appears on the microtuber slices if a successful integration of the GUS gene was achieved. From each explant 3 to 5 independent shoots were regenerated depending on the variety used. In all experiments the efficiency of positive shoots per explants is stated and the figures in table 2 have to be multiplied 3 to 5 times, yielding a transformation efficiency of up to 500%. Compared with selection systems used previously in potato plants the big advantage of the invention described is that together with a very high efficiency the number of shoots per explant is very high. For all four varieties transformed an extremely high transformation efficiency of up to 93% to 100% of all shoots regenerated was achieved at an Imazamox concentration of 0.5 µM. The experiment using 0.3 µM of Imazamox for selection shows how important the Imazamox concentration is for an efficient transformation rate. This can be seen from the results for variety Kuras in which a transformation efficiency of 7% at 0.3 µM Imazamox is increased to 93% at the 0.5 µM level.

**Table 2: Transformation efficiency of four different varieties with 0.3 and 0.5 µM Imazamox used as the selective agent.**

| **Variety** | **Construct** | **Selection Imazamox** **(µM)** | **Selection Imazamox** **(mg/l)** | **Number analysed** | **Number of positive** | **% positive** |
|---|---|---|---|---|---|---|
| Desiree | pAHASGUS | 0.3 µM | 0.0915mg/l | 19 | 12 | 63 |
| Prevalent | pAHASGUS | 0.3 µM | 0.0915mg/l | 26 | 16 | 62 |
| Producent | pAHASGUS | 0.3 µM | 0.0915mg/l | 25 | 19 | 76 |
| Kuras | pAHASGUS | 0.3 µM | 0.0915mg/l | 58 | 4 | 7 |
| | | | | | | |
| Prevalent | pAHASGUS | 0.5 µM | | 23 | 23 | 100 |
| | | | 0.1525mg/l | | | |
| Producent | pAHASGUS | 0.5 µM | | 40 | 38 | 95 |
| | | | 0.1525mg/l | | | |
| Kuras | pAHASGUS | 0.5 µM | | 33 | 32 | 97 |
| | | | 0.1525mg/l | | | |
| Desiree | pAHASGUS | 0.5 µM | 0.1525mg/l | 29 | 27 | 93 |

### Example 10

In a similar experiment compared to example 9 the *nptII* gene was used as selection marker being controlled by the same promoter nos as in pAHASGUS. The shoots are selected on 50 mg/l kanamycin, which is a standard kanamycin concentration used for potato transformation as described in Ooms, G. et al., 1987 and Tavazza, R. et al., 1988. The transformation frequency was much lower compared to when using a mutated AHAS gene e.g. the S653N gene as selection marker. The highest transformation efficiency based on analysis of the same GUS gene driven by the same GBSS promoter resulted in 74% transformed shoots regenerated.

### Example 11

The successful use of a mutated AHAS gene as a selection marker has been demonstrated using the promoter from the wild-type *Arabidopsis thaliana* AHAS gene (pAP1 construct) as well as a recombinant nos promoter (pAP2 construct) in the potato variety Seresta, see table 3. This shows that any promoter with sufficient expression can be used in connection with the mutated AHAS gene and thus the invention could be used with different promoters as regulatory elements for the mutated AHAS gene successfully yielding selection of transgenic shoots on selective medium. The AHAS gene can be driven by different promoters such as e.g. the AHAS-, nos-, 35S-, Rubisco- or vATPase - promoter.

**Table 3: Transformation efficiency of the mutated AHAS gene driven by two different promoters: Arabidopsis thaliana (A.t.) AHAS promoter and the nos promoter.**

| **Variety** | **Construct** | **Promoter** | **Number shoots analysed** | **Number shoots positive** | **% positive** |
|---|---|---|---|---|---|
| Seresta | pAP1 | A.t AHAS | 50 | 49 | 98 |
| Seresta | pAP2 | nos | 120 | 117 | 98 |

### Example 12

The mutated AHAS gene conferring resistance to Imazamox was used as a selection marker for the transformation of a commercially important trait to *Solanum tuberosum* (variety Seresta) resulting in potato lines producing amylopectine type starch. The starch component amylose is synthesized by granule-bound starch synthase (GBSS). Inhibition of the gene coding for GBSS directs starch production completely to amylopectin. An antisense gene fragment inhibiting the expression of gbss as described in EP-A 0 563 189 was co-transformed with the mutated AHAS gene conferring resistance to Imazamox.

The gbss promoter was used to drive the gbss antisense gene fragment. The Nos promoter or the AHAS promoter was used to drive the AHAS selection gene and resulted in a total transformation efficiency per shoots analysed of 98 to 100%, see table 4.

For restriction map of the T-DNA of the pAP1 and pAP2 constructs used, see figure 1 and 2. There were 3 to 5 independent shoots produced per explant yielding a transformation efficiency of 280 to 480% or up to 500% per explant. There were no differences in transformation efficiency between the different promoters and *Solanum tuberosum* varieties used.

**Table 4: pAP1 and pAP2 transgenic lines analysed for transformation efficiency and high amylopectin quality.**

| **Experiment** | **Variety** | **Construct** | **Number shoots analysed** | **Number shoots positive** | **% positive** | **high amylopectin content** |
|---|---|---|---|---|---|---|
| 1 | Seresta | pAP2 | 120 | 117 | 98 | + |
| 2 | Seresta | pAP1 | 50 | 49 | 98 | + |
| 3 | Seresta | pAP2 | 30 | 30 | 100 | + |
| 4 | Seresta | pAP2 | 3 | 3 | 100 | + |

Starch quality analysis was done with microtubers of the positive shoots with high quality integration of the intended genetic insert. The starch was mixed with iodine and the shoots exclusively containing amylopectin stains red-brown compared to the starch containing amylose that stains blue.

### References

Hawkes, T.R., in Prospects for Amino Acid Biosynthesis Inhibitors in crop protection and Pharmaceutical Chemistry, pp131-138, Brittish Crop Protection Council Monograph No 42, Surrey, U.K. (1989)
Herrera-Estrella, L., Depicker, a., Van Montagu, M., Schell, J., Expression of chimaeric genes transferred into plant cells using Ti-plasmid-derived vector, Nature 303:209-213 (1983)
Becker, D., Kemper, E., Schell, J., Masterson, R., New plant binary vectors with selectable markers located proximately to the left T-DNA border, Plant Molecular Biology 20:1195-1197, (1992)
Jefferson, R.A., Kavanagh, T.A., Bevan, M.W., GUS fusions: b-glucuronidase as a sensitive and versatile gene fusion marker in higher plants, EMBO Journal 6: 3901-3907 (1987)
Joersbo, M., Donaldson, I., Kreiberg, J., Guldager Petersen, S., Brunstedt, J., and Okkels, F.T., Analysis of mannose selection used for transformation of sugar beet, Molecular Breeding 4:111-117 (1998)
Libiakova, G., Jorgensen, B., Palmgren, G., Ulskov, P., Johansen, E., Efficacy of an intron containing kanamycin resistance gene as a selectable marker in plant transformation, Plant Cell Rep 20:610-615(2001)
Ooms, G., Burell, M.M., Karp, A., Bevan, M., Hille, J., Genetic transformation in two potato cultivars with T-DNA from disarmed Agrobacterium, Theoretical and Applied Genetics (1987) 73:744-750,
Ray, T.B., Plant Physiology (1984), 75, 827-831
Sathasivan, K., Haughn, G.W., Murai, N., Plant Physiology 97(1991), 1044-1050
Shaner, D.L., Andersson, P.C., Stidham, M.A., Plant Physiology 76 (1984), 545-546
Subrimanian, M.V., Gerwick, B.C., in Biocatalysis in Agricultural Biotechnology, pp277-288, ACS Symposium Series No389, American Chemical Society, Washington DC (1989)
Tavazza, R., Tavazza, M., Ordas, R.J., Ancora, G., Benvenuto, E., Genetic transformation of potato (Solanum tuberosum): An efficient method to obtain transgenic plants, Plant Science, 59 (1988), 175-181
Umbarger, H.E., in Synthesis of Amino Acids and Proteins, (1975) pp1-56, MTP International Review of Science, Butterworth, London.
Wesley S.V., Helliwell C.A., Smith N.A., Wang M.B., Rouse D.T., Liu Q., Gooding P.S., Singh S.P., Abbott D., Stoutjesdijk P.A., Robinson S.P., Gleave A.P., Green A.G., Waterhouse P.M., Construct design for efficient, effective and high-throughput gene silencing in plants, Plant J 2001 Sep;27(6):581-90
Haldrup A, Petersen, GP, Okkels FT (1998) The xylose isomerase gene from Thermoanaerobacterium thermosulfurogenes allows effective selection of transgenic plant cells using D-xylose as the selection agent. Plant Molecular Biology 37:287-296

## Claims

1. A method for transformation of potato plants by transforming potato plant cells with an expression vector comprising
regulatory sequences of a promoter active in plants, wherein the AHA synthase promoter or the nos promoter is used,
operably linked thereto a DNA sequence encoding a protein with the biological activity of an AHA synthase resistant to inhibitors of potato plant wildtype AHA synthase selected from the group of
a) a DNA sequence encoding a protein having SEQ-ID-No. 2 or chemically equivalent sequences of the protein,
b) a DNA sequence encoding a protein coded by SEQ-ID-No. 1,
c) a DNA sequence comprising a nucleotide sequence which hybridizes to a complementary strand of the nucleotide sequences a) or b) and
d) a DNA sequence being a derivative, analogue or fragment of a nucleotide sequence of a),b) or c) and encoding a protein possessing AHA synthase activity and conferring resistance to AHA synthase inhibitors,
operably linked thereto regulatory sequences which serve as transcription termination and/or polyadenylation signals in plants,
selecting for AHA synthase inhibitor resistant cells and regenerating them to transgenic plants.

2. A method for transformation according to claim 1, wherein for selection (RS)-2-(4-isopropyl-4-methyl-5-oxo-2-imidazolin- 2-yl)-5-methoxymethylnicotinic acid is used.

3. A transgenic potato plant cell produced by the method for transformation according to any of claims 1 to 2, wherein the regenerated plant exhibits an elevated resistance to imidazolinone type herbicides.

4. The transgenic potato plant cell according to claim 3, wherein the regenerated plant exhibits an elevated resistance to (RS) -2- (4-isopropyl-4-methyl-5-oxo-2-imidazolin- 2-yl) -5-methoxymethylnicotinic acid.

5. Tuber of the transgenic potato plant according to claims 3 or 4.

6. Use of a plant expression vector as **characterized in** claim 1 for selecting transgenic potato plant cells, potato tissue cultures and/or potato plants.
